# EUROPÄISCHE PATENTSCHRIFT

(11) **EP 0 728 722 B1**
(45) Veröffentlichungstag und Bekanntmachung des Hinweises auf die Patenterteilung: **06.05.1999**
(21) Anmeldenummer: 96101799.3
(22) Anmeldetag: 08.02.1996
(51) Int. Cl.: C07C 29/149, C07C 31/133

(54) **Verfahren zur Herstellung von Hydroxymethyl-cyclopropan**
Process for the production of hydroxymethylcyclopropane
Procédé pour la production d'hydroxyméthylcyclopropane

(30) Priorität: 21.02.1995 DE 19505939
(43) Veröffentlichungstag der Anmeldung: 28.08.1996
(73) Patentinhaber: BAYER AG, 51368 Leverkusen (DE)
(72) Erfinder: Frohn, Lutz, Dr., D-40699 Erkrath (DE); Langer, Reinhard, Dr., D-47800 Krefeld (DE); Darsow, Gerhard, Dr., D-47804 Krefeld (DE); Zirngiebl, Eberhard, Dr., D-51061 Köln (DE); Jentsch, Jörg-Dietrich, Dr., D-45468 Mülheim a.d. Ruhr (DE); Pennemann, Bernd, Dr., D-51061 Köln (DE); Tiburtius, Christoph, Dr., D-50999 Köln (DE)

(56) Entgegenhaltungen:
- EP-A- 0 222 988

## Beschreibung

Der Gegenstand der vorliegenden Erfindung ist ein Verfahren zur Herstellung von Hydroxymethyl-cyclopropan (Cyclopropyl-methanol) durch Hydrierung von Alkylestern der Cyclopropancarbonsäure in Gegenwart eines Zinkoxid enthaltenden chromfreien Katalysators.

Hydroxymethyl-cyclopropan findet Anwendung als Synthesebaustein bei der Herstellung von Arznei- und Pflanzenschutzmitteln.

Aus US 4.720.597 ist es bekannt, Hydroxymethyl-cyclopropan durch katalytische Hydrierung von Cyclopropancarbonsäureestern zu erhalten. Dieses Verfahren ergibt zwar wesentlich verbesserte Ausbeuten, verglichen mit Verfahren des in diesem US-Patent genannten Standes der Technik; es ist jedoch erforderlich, einen Zn-Cr-Katalysator einzusetzen, der durch die hohe Giftigkeit der verwendeten Cr-Verbindung gekennzeichnet ist.

Es bestand daher der Wunsch, ein Hydrierverfahren zur Herstellung von Hydroxymethyl-cyclopropan aus Cyclopropancarbonsäureestern zu entwickeln, das sowohl wirtschaftlich als auch umweltfreundlich ist und insbesondere ohne hochgiftige Cr-Verbindungen durchführbar ist. Es wurde nun überraschend gefunden, daß beide Zielsetzungen durch den Einsatz eines Zn-haltigen Katalysators erreicht werden können, bei dem auf das giftige Cr verzichtet werden kann.

Die Erfindung betrifft ein Verfahren zur Herstellung von Hydroxymethyl-cyclopropan aus Cyclopropancarbonsäure-C₁-C₁₀-alkylestern, das dadurch gekennzeichnet ist, daß man die Cyclopropancarbonsäureester in Gegenwart eines Zinkoxid enthaltenden, chromfreien Katalysators bei 50 bis 350 bar und 150 bis 350°C mit überschüssigem Wasserstoff hydriert.

Cyclopropancarbonsäure-alkylester für das erfindungsgemäße Verfahren sind solche, in denen der Alkylrest 1 bis 10 C-Atome aufweist, beispielsweise Methyl, Ethyl, Propyl, Isopropyl, Butyl, Isobutyl, Pentyl, Hexyl, Octyl, Decyl, bevorzugt C₁-C₈-Alkylreste der genannten Art, besonders bevorzugt C₁-C₄-Alkylreste der genannten Art.

Der Druckbereich für das erfindungsgemäße Verfahren umfaßt 50 bis 350 bar, bevorzugt 180 bis 350 bar, besonders bevorzugt 190 bis 300 bar.

Der Temperaturbereich für das erfindungsgemäße Verfahren umfaßt 150 bis 350°C, bevorzugt 250 bis 325°C, besonders bevorzugt 270 bis 325°C.

Es ist möglich, das erfindungsgemäße Verfahren ohne Verwendung eines Lösungsmittels oder Lösungsmittelgemisches durchzuführen, jedoch ist auch die Anwesenheit eines unter den Reaktionsbedingungen stabilen Lösungsmittels oder Lösungsmittelgemisches möglich, wobei dem Fachmann für solche Anwendungen geläufige Lösungsmittel eingesetzt werden können. Da die Anwesenheit solcher Lösungsmittel die Aufarbeitung erschwert, wird bevorzugt ohne Gegenwart solcher Lösungsmittel gearbeitet.

Es ist erfindungsgemäß möglich, etwaige Destillationsrückläufe, beispielsweise mit einem Gehalt an nicht vollständig hydrierten Cyclopropancarbonsäureestern, in die Reaktion zurückzuführen.

Das erfindungsgemäße Verfahren kann sowohl diskontinuierlich als auch kontinuierlich durchgeführt werden. Die zur Durchführung des Verfahrens erforderlichen Apparaturen sind im Stand der Technik bekannt und dem Fachmann geläufig. Beispielsweise seien genannt: Zweiphasenreaktoren mit fest angeordnetem oder bewegtem Katalysatorbett, wobei der Katalysator die feste Phase bildet und die anderen Komponenten (Edukt, Produkte und H₂) sich in der Gasphase befinden, und Mehrphasenreaktoren, bei denen neben der festen und der gasförmigen Phase eine oder mehrere flüssige Phasen vorliegen, wie z.B. begaster Rührkessel, Blasensäule, Sumpfphasenreaktor oder Rieselphasenreaktor. Besonders vorteilhaft läßt sich das erfindungsgemäße Verfahren kontinuierlich ausführen. Dies erfolgt in vorteilhafter Weise dadurch, daß man das flüssige Ausgangsmaterial über den im Reaktor befindlichen, stückig angeordneten Katalysator, beispielsweise nach dem Rieselphasenprinzip, führt, während der Wasserstoff im Gleich- oder Gegenstrom durch das Reaktionsrohr geleitet wird. Vorteilhafterweise wird der im Überschuß eingesetzte Wasserstoff (1- bis 500-molarer Überschuß) im Kreis geführt. Besonders bevorzugt wird das erfindungsgemäße Verfahren in der Gasphase durchgeführt. Dem Fachmann sind vergleichbare Hydrierverfahren und ihre Durchführung im Abhängigkeit von der Wahl der Bedingungen, beispielsweise der Temperatur, dem Druck und dem Molverhältnis Edukt : Wasserstoff bekannt.

Das erfindungsgemäße Verfahren ist gekennzeichnet durch den Einsatz eines Zinkoxid enthaltenden chromfreien Katalysators. Das Zinkoxid kann durch jede dem Fachmann bekannte Art und Weise, beispielsweise durch Verbrennen von metallischem Zink oder durch Behandlung wäßriger Lösungen von Zinksalzen mit Basen erhalten werden. Ein so gefälltes Zinkoxid wird getrocknet. Das Zinkoxid kann sowohl in der erhaltenen Form als auch nach einem oder mehreren weiteren Verarbeitungsschritten eingesetzt werden. So wird für den Einsatz in Suspensionsphasen das Zinkoxid in Pulverform bevorzugt, während es für Riesel- und Gasphasenreaktoren vorteilhaft ist, das Zinkoxid in stückige Form zu bringen. Hierbei wird beispielsweise der durch Fällung von Zinksalz-Lösungen erhaltene feuchte Niederschlag in einem Kneter durchgeknetet und in einem Granulierapparat zu Formlingen verarbeitet. Die noch feuchten Formkörper werden anschließend bei 300 bis 500°C während 1 bis 10 Stunden kalziniert. Gleichermaßen kann der Zinkoxid-Katalysator aber auch auf einen dem Fachmann bekannten Träger, wie SiO₂, Al₂O₃, TiO₂, ZrO₂, MgO usw. aufgezogen, oder mit Hilfe eines Bindemittels (Tablettierhilfsmittels) verpreßt und granuliert werden. Unter Berücksichtigung des Einsatzes von reinem Zinkoxid (100 %) beträgt der Anteil von ZnO im Katalysator 20-100 %, bevorzugt 50-100 % des Katalysator-Gesamtgewichts. Die Menge des Tablettierhilfsmittels, z.B. Graphit, beträgt 0-20 % des Katalysator-Gesamtgewichts unter Berücksichtigung seiner Nichtanwendung (0 %).

Zinkoxid ist bezüglich des erfindungsgemäßen Verfahrens eine aktive Substanz, deren Aktivität auch durch Vermischen mit anderen Feststoffen erhalten, modifiziert oder gesteigert werden kann. Zur Erhöhung der Aktivität dieses Katalysators kann es vorteilhaft sein, weitere Komponenten, wie beispielsweise Verbindungen von Natrium, Kalium, Magnesium, Calcium, Vanadium, Niob, Tantal, Molybdän, Eisen, Ruthenium, Kobalt, Rhodium, Nickel, Palladium, Platin, Rhenium, Kupfer oder Silber in einer Menge von 1-30 g Atom-% zuzusetzen, gerechnet als der Komponente zugrundeliegendes Metall und bezogen auf g-Atommenge Zn. Die Aufbringung kann dabei in jeder dem Fachmann bekannten Weise, beispielsweise durch gleichzeitiges oder getrenntes Fällen und nachträgliches Zusammenbringen oder durch Tränken des Zinkoxids mit in Wasser oder anderen Flüssigkeiten gelösten Verbindungen und anschließendes Trocknen geschehen.

Zusätze von Kupfer haben sich besonders bewährt.

In bevorzugter Weise wird reines Zinkoxid mit einer inneren Oberfläche von mehr als 10 m²/g (BET), besonders bevorzugt mehr als 35 m²/g und bis zu 150 m²/g, gegebenenfalls auf einem Träger oder verdünnt mit Inertmaterialien, eingesetzt.

### Beispiele

### Beispiel 1

Durch ein Reaktionsrohr, gefüllt mit 40 ml ZnO-Katalysator (Tabletten, 5x3 mm), wurden stündlich 4 g Cyclopropancarbonsäure-methylester und 80 Nl Wasserstoff geführt. Die Temperatur betrug 297°C und der Druck 300 bar.

Der Umsatz an Cyclopropancarbonsäuremethylester betrug 99 %, die Selektivität zum Hydroxymethyl-cyclopropan 92 %.

### Beispiel 2

Ausführung wie in Beispiel 1, jedoch wurde Cyclopropancarbonsäure-ethylester eingesetzt.

Der Umsatz an Cyclopropancarbonsäure-ethylester betrug 99 %, die Selektivität zum Hydroxymethyl-cyclopropan 93 %.

### Beispiel 3

Ausführung wie in Beispiel 1, jedoch wurde Cyclopropancarbonsäure-propylester eingesetzt.

Der Umsatz an Cyclopropancarbonsäure-propylester betrug 98 %, die Selektivität zum Hydroxymethyl-cyclopropan 90 %.

### Beispiel 4

Ausführung wie in Beispiel 1, jedoch wurde Cyclopropancarbonsäure-butylester eingesetzt.

Der Umsatz an Cyclopropancarbonsäure-butylester betrug 99 %, die Selektivität zum Hydroxymethyl-cyclopropan 89 %.

### Beispiel 5

Ausführung wie in Beispiel 1, jedoch wurde Cyclopropancarbonsäure-isobutylester eingesetzt.

Der Umsatz an Cyclopropancarbonsäure-isobutylester betrug 99 %, die Selektivität zum Hydroxymethyl-cyclopropan 93 %.

### Beispiel 6

Durch ein Reaktionsrohr, gefüllt mit 50 ml kupferdotierten ZnO-Tabletten (enthaltend 50 g Kupfer in Form von Kupferoxid und/oder Kupferhydroxid pro Liter ZnO-Tabletten), wurden stündlich 5,5 g Cyclopropancarbonsäure-isobutylester und 300 Nl Wasserstoff geführt. Die Temperatur betrug 207°C, der Druck 300 bar.

Der Umsatz an Cyclopropancarbonsäure-isobutylester betrug 98 %, die Selektivität zum Hydroxymethyl-cyclopropan 91 %.

## Patentansprüche

1. Verfahren zur Herstellung von Hydroxymethyl-cyclopropan aus Cyclopropancarbonsäure-C₁-C₁₀-alkylestern, dadurch gekennzeichnet, daß man die Cyclopropancarbonsäureester in Gegenwart eines Zinkoxid enthaltenden, chromfreien Katalysators bei 50 bis 350 bar und 150 bis 350°C mit überschüssigem Wasserstoff hydriert.

2. Verfahren nach Anspruch 1, dadurch gekennzeichnet, daß man bei 180 bis 350 bar hydriert.

3. Verfahren nach Anspruch 1, dadurch gekennzeichnet, daß man bei 250 bis 325°C hydriert.

4. Verfahren nach Anspruch 1, dadurch gekennzeichnet, daß ein Cyclopropancarbonsäure-C₁-C₈-alkylester, bevorzugt ein Cyclopropancarbonsäure-C₁-C₄-alkylester eingesetzt wird.

5. Verfahren nach Anspruch 1, dadurch gekennzeichnet, daß man Zn-Oxid als Katalysator einsetzt.

6. Verfahren nach Anspruch 1, dadurch gekennzeichnet, daß die Hydrierung in der Gasphase oder Rieselphase durchgeführt wird, wobei der Wasserstoff im Gleich- oder Gegenstrom geführt wird.

7. Verfahren nach Anspruch 6, dadurch gekennzeichnet, daß die Hydrierung in der Gasphase durchgeführt wird.

8. Verfahren nach Anspruch 1, dadurch gekennzeichnet, daß man Zn-Oxid mit einem Kupferzusatz als Katalysator einsetzt.

## Claims

1. Process for the preparation of hydroxymethylcyclopropane from cyclopropanecarboxylic acid C₁-C₁₀-alkyl esters, characterized in that the cyclopropanecarboxylic acid esters are hydrogenated with excess hydrogen in the presence of a chromium-free catalyst comprising zinc oxide under 50 to 350 bar at 150 to 350°C.

2. Process according to Claim 1, characterized in that the hydrogenation is carried out under 180 to 350 bar.

3. Process according to Claim 1, characterized in that the hydrogenation is carried out at 250 to 325°C.

4. Process according to Claim 1, characterized in that a cyclopropanecarboxylic acid C₁-C₈-alkyl ester, preferably a cyclopropanecarboxylic acid C₁-C₄-alkyl ester, is employed.

5. Process according to Claim 1, characterized in that Zn oxide is employed as the catalyst.

6. Process according to Claim 1, characterized in that the hydrogenation is carried out in the gas phase or trickle phase, the hydrogen being passed in co- or countercurrent.

7. Process according to Claim 6, characterized in that the hydrogenation is carried out in the gas phase.

8. Process according to Claim 1, characterized in that Zn oxide with an addition of copper is employed as the catalyst.

## Revendications

1. Procédé pour la préparation de l'hydroxyméthylcyclopropane à partir d'esters alkyliques en C₁-C₁₀ de l'acide cyclopropanecarboxylique, caractérisé en ce que l'on hydrogène l'ester cyclopropanecarboxylique en présence d'un catalyseur contenant de l'oxyde de zinc mais exempt de chrome à des pressions de 50 à 350 bar et des températures de 150 à 350°C par un excès d'hydrogène.

2. Procédé selon revendication 1, caractérisé en ce que l'on hydrogène à des pressions de 180 à 350 bar.

3. Procédé selon revendication 1, caractérisé en ce que l'on hydrogène à des températures de 250 à 325°C.

4. Procédé selon revendication 1, caractérisé en ce que l'on met en oeuvre un cyclopropanecarboxylate d'alkyle en C₁-C₈, de préférence un cyclopropanecarboxylate d'alkyle en C₁-C₄.

5. Procédé selon revendication 1, caractérisé en ce que le catalyseur consiste en oxyde de zinc.

6. Procédé selon revendication 1, caractérisé en ce que l'hydrogénation est réalisée en phase gazeuse ou en phase ruisselante avec passage de l'hydrogène en courant parallèle ou à contre-courant.

7. Procédé selon revendication 6, caractérisé en ce que l'hydrogénation est réalisée en phase gazeuse.

8. Procédé selon revendication 1, caractérisé en ce que l'on utilise en tant que catalyseur un oxyde de zinc additionné de cuivre.
